# NEW EUROPEAN PATENT SPECIFICATION

(11) **EP 0 541 279 B2**
(45) Date of publication and mention of the opposition decision: **02.01.2002**
(45) Mention of the grant of the patent: 17.12.1997
(21) Application number: 92309766.1
(22) Date of filing: 26.10.1992
(51) Int. Cl.: G05B 19/10, A61M 5/172

(54) **Electronically controlled infusion devices and arrangements**
Elektronisch gesteuerte Infusionsvorrichtung
Dispositif de perfusion à commande électronique

(30) Priority: 04.11.1991 GB 9123325
(43) Date of publication of application: 12.05.1993
(73) Proprietor: BAXTER INTERNATIONAL INC., Deerfield, IL 60015-4633 (US)
(72) Inventor: Danby, Hal, Sudbury, Suffolk CO10 0PZ (GB)
(74) Representative: Dee, Ian Mark

(56) References cited:
- EP-A- 0 335 161
- DE-A- 2 757 593
- US-A- 4 396 385
- US-A- 5 115 509

## Description

This invention relates to electronically controlled infusion devices and arrangements and more particularly to infusion controllers or infusion pump arrangements which may be set to provide a desired volume of fluid to be infused at a desired flow rate as dictated by the needs of a patient under treatment and requiring fluids to be infused intravenously.

Examples of such pump arrangements are to be found described in GB-2225065A.

As is well-known, medical staff operate under considerable time pressure and one object of the present invention is to provide improved infusion controllers and volumetric infusion pump arrangements which may be set to provide required volumes of liquid to be infused and/or rates of flow relatively speedily and in a manner which tends to reflect the sense of urgency with which medical staff operate.

Conventional electronically controlled intravenous infusion apparatus commonly include a manually operable control knob which is rotated by the operator to set at least one infusion parameter of the apparatus according to the extent to which the knob is rotated.

According to the present invention, there is provided an electronically controlled intravenous infusion apparatus according to claim 1.

Preferably, the apparatus is such that for setting the flow rate said sensing means is responsive to two rates of movement, normal or slow movement and fast movement.

Preferably the apparatus is such that for setting the volume-to-be-infused said sensing means is responsive to three rates of movement.

Preferably the apparatus is such that for setting the volume-to-be-infused said sensing means is responsive to three rates of movement, normal or slow movement causing incremental setting changes; fast movement causing the setting to jump to the next multiple of 100ml of volume of fluid to be infused and very fast movement causing the setting to jump to the next adjacent multiple of 1000ml of volume-to-infused.

Preferably said movement is rotary movement and preferably said control device is a digipot or shaft encoder

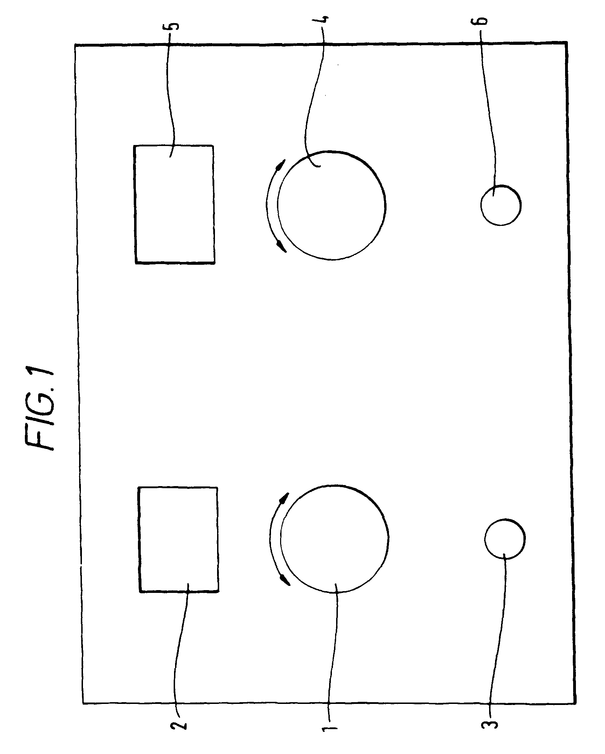

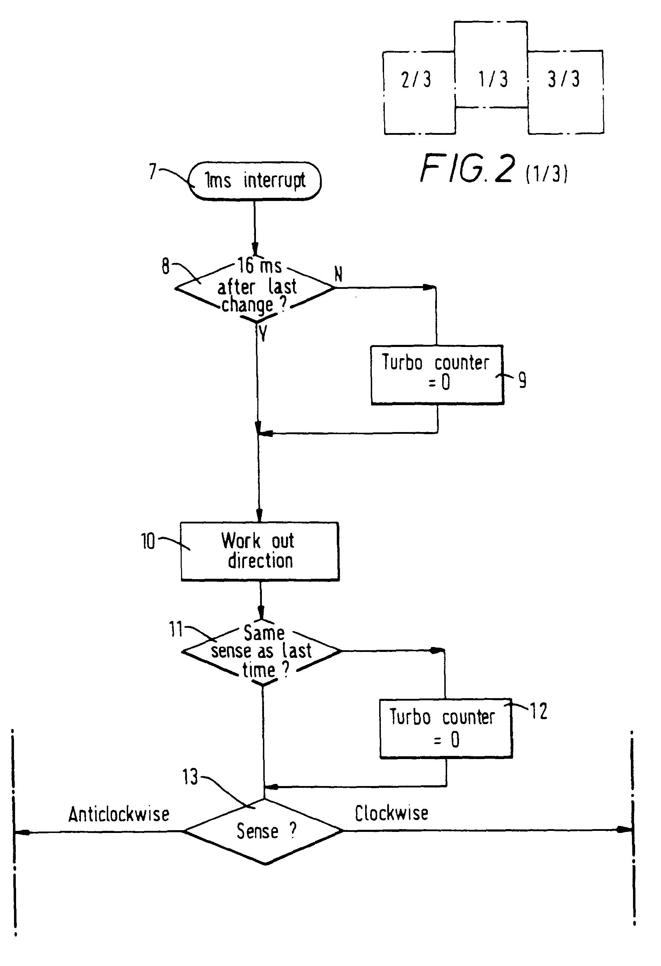

The invention is illustrated in and further with reference to the accompanying drawings in which:-
Figure 1 is a schematic representation of the control panel of one example of electronically controlled volumetric infusion pump arrangement in accordance with the present invention.
Figure 2 is a flow chart appertaining to the microprocessor program controlling the flow rate setting of the pump and
Figure 3 is a flow chart appertaining to the microprocessor program controlling the volume-to-be-infused setting of the pump.

Referring to the drawings, the microprocessor controlled pump (not represented) is as described in detail in our U.K. specification published under serial number 2225065A to which reference may be made for further information.

Setting of the flow rate of the pump is provided for by means of a so-called digipot or shaft encoder 1. A digital display 2 is arranged to indicate the flow rate setting achieved as digipot 1 is rotated clockwise to increase the rate or anti-clockwise to decrease the rate. A "confirm" button 3 is arranged, when pressed, to cause the pump to commence pumping at the selected rate, when a "start" button (not shown) is operated, or to change from a previously selected rate to the selected new rate, as displayed on display 2.

Adjustment of the volume-to-be-infused setting of the pump is provided for by means of another digipot 4. A digital display 5 is arranged to display the volume-to-be-infused setting achieved as digipot 4 is rotated clockwise to increase the volume-to-be-infused or anti-clockwise to decrease the volume-to-be-infus ed. A "confirm" button 6 is arranged, when pressed, to cause the pump to deliver the selected volume of fluid (at the flow-rate set by digipot 1) when the aforementioned start button is operated. If the pump is already running, operating "confirm" button 6 causes the selected volume-to-be-infused setting to be changed to the new setting, as selected by digipot 4.

Digipots 1 and 4 each have sixteen detented positions in three hundred and sixty degrees. The pump is required to be programmed to provide flow rates of between 0.5 and 999ml/hr and to deliver volumes of between 1 and 9999ml. Relying upon a normal change of one digipot position per unit change in flow rate or volume-to-be-infused, sweeping through the whole range in each case would be time consuming in the extreme.

However, the microprocessor is programmed to be responsive to the rate at which pulses are generated by the digipots 1 or 4 such that upon fast rotation the setting of the respective operating parameter (flow rate or volume-to-be-infused, as the case may be) jumps from one predetermined preferred setting to the next (greater or lesser dependant upon the direction of rotation) in a series of preferred settings ("sticky numbers"). From that setting, the respective digipot is rotated again either quickly to jump to the next adjacent predetermined preferred setting or slowly to produce a change of setting unit by unit as the digipot in rotated from detented position to detented position.

For setting the flow rate, the preferred rates between which the settings jump upon fast rotation of the digipot 1, in ml/hr are:-

| |
|---|
| 0.5 |
| 5.0 |
| 25 |
| 41 |
| 50 |
| 81 |
| 100 |
| 125 |

and thereafter in multiples of 100ml/hr up to the aforementioned 999ml/hr.

For setting the flow rate, all of the preferred settings have the same priority For setting the volume-to-be-infused however, it is more likely that multiples of 1000ml will be selected as the volume required rather than some intermediate value. Taking this into account, in the case of the volume-to-be-infused digipot 4 the microprocessor is programmed to be responsive to a third speed band as follows:-
- Slow rotation: One increment/decrement per digipot detent position.
- Fast rotation: Jump to next adjacent larger/smaller multiple of 100ml for one (or more) digipot detent position.
- Very fast rotation: (flicking) Change to next adjacent larger/smaller multiple of 1000ml.

Provided that the number of volume-to-be-infused digipot detent positions traversed does not exceed the number of positions that would be required to be traversed under slow rotation to change from one preferred setting to the next, on fast or very fast (flicking) rotation the number of detent positions traversed is irrelevant.

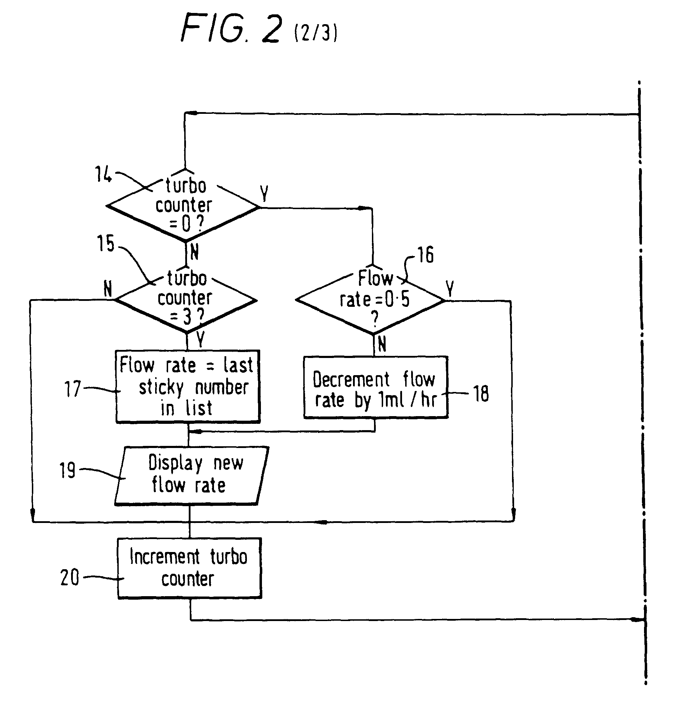

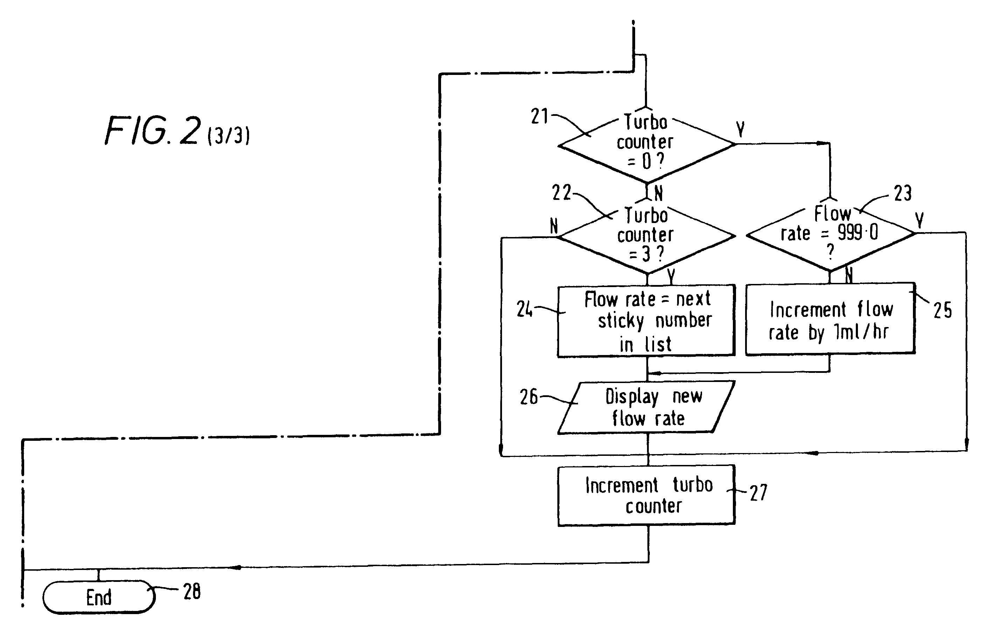

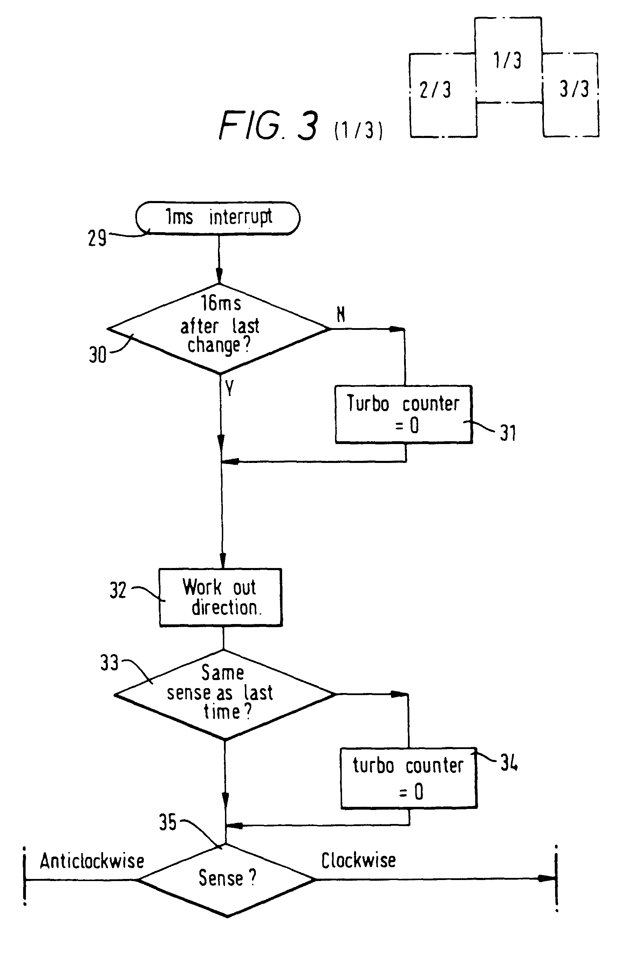

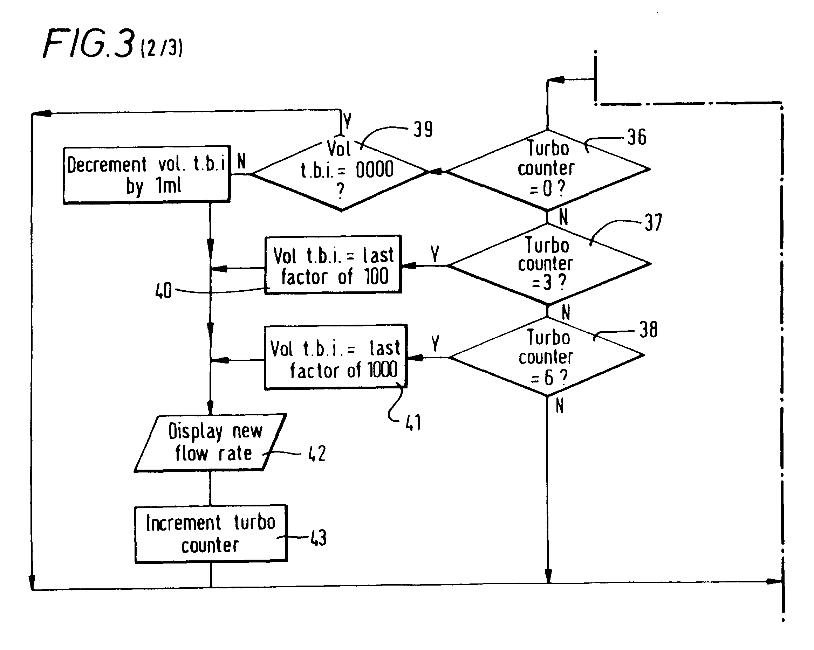

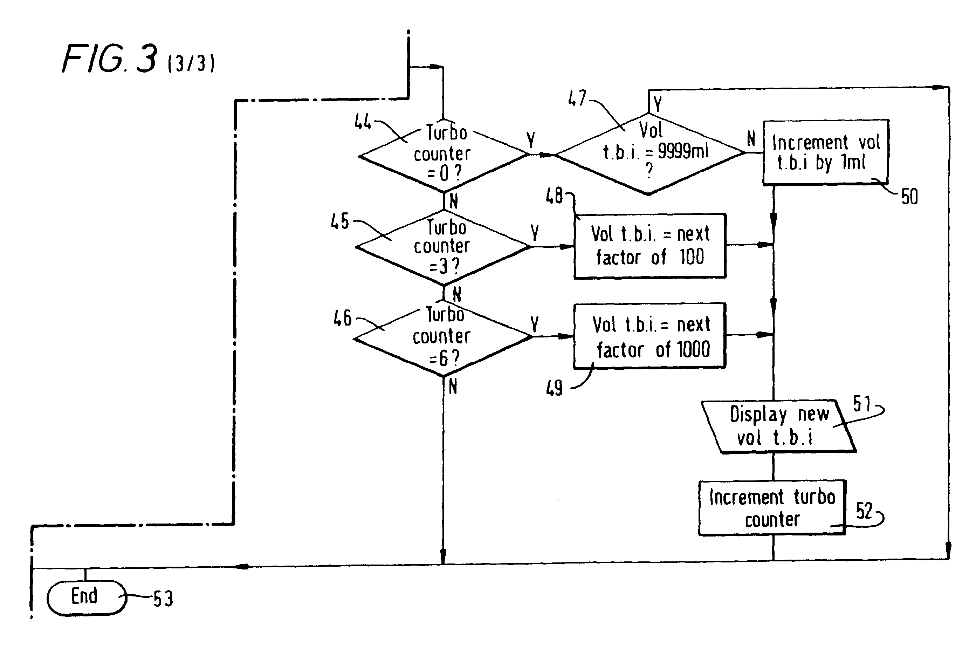

Referring to Figure 2 and 3, whilst the programming of the microprocessor will be a matter within the competance of one skilled in the art having regard to the information hereinbefore given, the flow chart of Figure 2 appertaining to flow rate setting and the flow chart of Figure 3 appertaining to volume-to-be-infused (Vol t.b.i.) setting may be of assistance in this. For the sake of clarity, the legends used in the different blocks are repeated below.

### Figure 2

7. "1ms interrupt"
8. "16ms after last change?"
9. "Turbo counter = 0"
10. "Work out direction"
11. "Same sense as last time?"
12. "Turbo counter = 0"
13. "Sense?"
14. "Turbo counter = 0?"
15. "Turbo counter = 3?"
16. "Flow rate = 0.5?"
17. "Flow rate = last sticky number in list"
18. "Decrement flow rate by 1ml/hr"
19. "Display new flow rate"
20. "Increment turbo counter"
21. "Turbo counter = 0?"
22. "Turbo counter = 3?"
23. "Flow rate = 999.0?"
24. "Flow rate = next sticky number in list"
25. "Increment flow rate by 1ml/hr"
26. "Display new flow rate"*£12)
27. "Increment turbo counter"
28. "End".

### Figure 3

29. "1ms interrupt"
30. "16ms after last change?"
31. "Turbo counter = 0"
32. "Work out direction"
33. "Same sense as last time?"
34 "Turbo counter = 0"
35. "Sense"
36. "Turbo counter = 0?"
37. "Turbo counter = 3?"
38. "Turbo counter = 6?"
39. "Vol. t.b.i. = 0000?"
40. "Vol. t.b.i. = last factor of 100"
41. "Vol. t.b.i. = last factor of 1000"
42. "Display new flow rate"
43. "Increment turbo counter"
44. "Turbo counter = 0?"
45. "Turbo counter = 3?"
46. "Turbo counter = 6"
47. "Vol t.b.i. = 9999ml?"
48. "Vol t.b.i. = next factor of 100"
49. "Vol t.b.i. = next factor of 1000"
50. "Increment vol t.b.i. by 1ml"
51. "Display new Vol t.b.i."
52. "Increment turbo counter"
53. "End".

## Claims

1. An electronically controlled intravenous infusion apparatus comprising setting means for setting the volume to be infused and the flow rate parameters of the apparatus in response to a respective signal generated by dedicated control devices (1,4) for each parameter, operatively connected with the setting means, each control device (1,4) comprising a movable control member and sensing means operable to sense the extent and speed that the control member is moved by an operator, each signal being indicative of the extent and speed of movement of said control member, the sensing means being operable to sense two or more rates of movement of the control member, including slow movement causing incremental change in the setting of the parameter, or respective parameter, and faster movement causing the setting of said parameter to jump from one predetermined preferred setting to another separated therefrom by a plurality of incremental setting changes.

2. Apparatus as claimed in Claim 1 and such that for setting the flow rate said sensing means is operable to sense slow movement and fast movement.

3. Apparatus as claimed in Claim 1 or Claim 2 and such that for setting the volume-to-be-infused said sensing means is operable to sense three rates of movement.

4. Apparatus as claimed in Claim 3 and such that the sensing means is operable to sense slow movement of the control member of the control device for setting the volume to be infused causing incremental setting changes; fast movement causing the setting to jump to the next multiple of 100ml of volume of fluid to be infused and very fast movement causing the setting to jump to the next adjacent multiple of 1000ml of volume-to-be-infused.

5. Apparatus as claimed in any of the above claims and wherein said movement is rotary movement.

6. Apparatus as claimed in any preceding claim wherein each said control device (1,4) is a digipot or shaft encoder.

## Patentansprüche

1. Elektronisch gesteuerte intravenöse Infusionsvorrichtung, die eine Einstelleinrichtung zum Einstellen des zu infundierenden Volumens und der Durchflußratenparameter der Vorrichtung in Abhängigkeit von einem jeweiligen Signal aufweist, die für jeden Parameter von speziellen Steuereinrichtungen (1, 4) erzeugt werden, die mit der Einstelleinrichtung betriebsmäßig verbunden sind, wobei jede Steuereinrichtung (1, 4) ein bewegbares Steuerelement und eine Abtasteinrichtung aufweist, die so ausgelegt sind, daß sie das Ausmaß und die Geschwindigkeit erfassen, mit denen das Steuerelement von einem Bediener bewegt wird, wobei jedes Signal das Ausmaß und die Geschwindigkeit der Bewegung des Steuerelementes anzeigt,
wobei die Abtasteinrichtung so betreibbar ist, daß sie zwei oder mehr Bewegungsraten des Steuerelementes erfaßt, einschließlich einer langsamen Bewegung, die eine inkrementale Änderung der Einstellung des Parameters oder des jeweiligen Parameters bewirkt, und einer schnelleren Bewegung, die bewirkt, daß die Einstellung des Parameters von einer vorbestimmten bevorzugten Einstellung zu einer anderen springt, die davon durch eine Vielzahl von inkrementalen Einstellungsänderungen getrennt ist.

2. Vorrichtung nach Anspruch 1,
die derart ausgebildet ist,
daß zum Einstellen der Durchflußrate die Abtasteinrichtung betätigbar ist, um eine langsame Bewegung und eine schnelle Bewegung zu erfassen.

3. Vorrichtung nach Anspruch 1 oder 2,
die derart ausgebildet ist,
daß zum Einstellen des zu infundierenden Volumens die Abtasteinrichtung so betätigbar ist, daß sie drei Bewegungsgeschwindigkeiten erfaßt.

4. Vorrichtung nach Anspruch 3,
die derart ausgebildet ist,
daß die Abtasteinrichtung betätigbar ist, um folgendes zu erfassen:
eine langsame Bewegung des Steuerelementes der Steuereinrichtung zum Einstellen des zu infundierenden Volumens, die inkrementale Einstelländerungen bewirkt;
eine schnelle Bewegung, die bewirkt, daß die Einstellung zu dem nächsten Vielfachen von 100 ml Volumen von zu infundierendem Fluid springt; und
eine sehr schnelle Bewegung, die bewirkt, daß die Einstellung zu dem nächsten benachbarten Vielfachen von 1000 ml von zu infundierendem Volumen springt.

5. Vorrichtung nach einem der vorhergehenden Ansprüche,
wobei die Bewegung eine Drehbewegung ist.

6. Vorrichtung nach einem der vorhergehenden Ansprüche,
wobei jede Steuereinrichtung (1, 4) ein Digitalpotentiometer oder ein Drehgeber ist.

## Revendications

1. Appareil de perfusion intraveineuse à commande électronique comprenant des moyens de réglage permettant de régler le volume à perfuser et les paramètres de débit de l'appareil en fonction d'un signal respectif généré par les dispositifs de commande associés (1,4) pour chaque paramètre, opérationnellement reliés aux moyens de réglage, chaque dispositif de commande (1,4) comprenant un élément de commande mobile et des moyens de détection pouvant être utilisés pour détecter l'ampleur et la vitesse du déplacement de l'élément de commande par un opérateur, chaque signal étant indicatif de l'ampleur et de la vitesse du mouvement dudit élément de commande, les moyens de détection pouvant être utilisés pour détecter deux ou plusieurs vitesses de mouvement de l'élément de commande, y compris un mouvement lent provoquant une modification incrémentielle du réglage du paramètre, ou du paramètre respectif, et un mouvement plus rapide provoquant l'augmentation rapide du réglage dudit paramètre passant d'une valeur de réglage préférée prédéterminée à une autre valeur séparée de la valeur précédente par une pluralité de modifications de réglage incrémentielles.

2. Appareil selon la revendication 1, tel que, pour le réglage du débit, lesdits moyens de détection puissent être utilisés pour détecter un mouvement lent ou un mouvement rapide.

3. Appareil selon les revendications 1 ou 2, tel que, pour le réglage du volume à transfuser, lesdits moyens de détection puissent être utilisés pour détecter trois vitesses de mouvement.

4. Appareil selon la revendication 3, tel que les moyens de détection puissent être utilisés pour détecter un mouvement lent de l'élément de commande du dispositif de commande pour le réglage du volume à transfuser, provoquant des variations de réglage incrémentielles, un mouvement rapide provoquant une augmentation du réglage à la valeur suivante multiple de 100 ml de volume de fluide à transfuser et un mouvement très rapide provoquant une augmentation du réglage à la valeur suivante multiple de 1000 ml de volume à transfuser.

5. Appareil selon l'une quelconque des revendications précédentes, dans lequel ledit mouvement est un mouvement rotatif.

6. Appareil selon l'une quelconque des revendications précédentes, dans lequel chaque dispositif de commande (1,4) est un potentiomètre digital ou un encodeur rotatif.
